# EUROPEAN PATENT SPECIFICATION

(11) **EP 3 793 438 B1**
(45) Date of publication and mention of the grant of the patent: **27.08.2025**
(21) Application number: 19802697.3
(22) Date of filing: 17.05.2019
(51) Int. Cl.: A61B 5/11, A61B 5/024, A61B 5/00, A63B 24/00

(54) **BODY SENSOR**
KÖRPERSENSOR
CAPTEUR CORPOREL

(30) Priority: 18.05.2018 FI 20185460
(43) Date of publication of application: 24.03.2021
(73) Proprietor: Corle OY, 00550 Helsinki (FI)
(72) Inventor: LEHTONEN, Peik, 00550 Helsinki (FI)
(74) Representative: Primrose Oy
(86) International application number: PCT/FI2019/050388
(87) International publication number: WO 2019/220020

(56) References cited:
- WO-A1-2015/189687
- TW-U- M 543 053
- US-A1- 2010 217 103
- US-A1- 2012 253 152
- US-A1- 2013 085 346
- US-A1- 2014 266 939
- US-A1- 2018 092 601
- US-A1- 2018 098 734
- MING-ZHER POH ET AL: "Motion-Tolerant Magnetic Earring Sensor and Wireless Earpiece for Wearable Photoplethysmography", IEEE TRANSACTIONS ON INFORMATION TECHNOLOGY IN BIOMEDICINE, IEEE SERVICE CENTER, LOS ALAMITOS, CA, US, vol. 14, no. 3, 1 May 2010 (2010-05-01), pages 786 - 794, XP011345704, ISSN: 1089-7771, DOI: 10.1109/TITB.2010.2042607

## Description

### FIELD OF THE INVENTION

The present invention relates to a body sensor for measuring body activity of a user as defined in the preamble of the independent claim 1.

### BACKGROUND OF THE INVENTION

In the prior art, different kinds of wrist-worn body sensors are known which measure for example heart rate activity from a user's wrist. Also, optical heart rate sensors are known which are used in the upper arm or forearm. However, these kinds of heart rate sensors that are placed in connection with user's arm or wrist do not suit for everyone and especially those having problems with their arms or those who use their arms extensively so that the wrist-worn heart rate sensors disturb their workout.

It is also known in the prior art that heart rate activity can be measured from the user's ear. WO 2017/189687 discloses an integrated device to calculate heart rate and body activity. The device consists of an accelerometer and a heart rate monitor within a miniature device placed over the ear. Such placement over the ear is done using magnets. The device includes a microcontroller, a vibrator and a wireless networking module, which allows it to communicate with other devices, such as smart phones, computers, etc. Heart rate is extracted using reflected photo plethysmography from behind the ear. The means of measuring this is done using infrared LEDs and photodiodes. The means for affixing the device onto the ear comprises a body containing a magnet and the same body is also fixed to a flexible material, which contains another magnet. In other words, the device disclosed in WO 2017/189687 is a one-piece device comprising two magnets which are connected to each other through the flexible material. The documents WO 2015/189687, US 2010/217103, TW M 543 053, US 2012/253152 as well as MINGH-ZHER POH ET AL: "Motion-Tolerant Magnetic Earring Sensor and Wireless Earpiece for Wearable Photoplethysmography" also disclose different kind of ear sensors.

One of the problems associated with the prior art is that with a one-piece device the placement of the device is limited and the device becomes larger and therefore heavier which is a downside when using the device in sports such as running or other hard-paced exercise. Another problem with the prior art is that strength of the magnetic connection between the two magnets cannot be changeable in cases where the ear size is different or the need for the intensity varies. Further a problem with the one-piece device is a risk that the device will stick to hair or other sports equipment or cloths.

### BRIEF DESCRIPTION OF THE INVENTION

An object of the present invention is to provide an effective body sensor which can be freely attached to the user's body. A further object of the present invention is to provide an effective ear-worn heart rate sensor which can be firmly attached to the user's ear.

The objects of the invention are achieved by a body sensor which is characterized by what is stated in the independent claim. The preferred embodiments of the invention are disclosed in the dependent claims.

The invention relates to a body sensor to be used in connection with a body part, for example in connection with a nose, a lip, an arm or an ear, and preferably in connection with an earlobe. The body sensor uses an optical sensor to measure user's body activity such as heart rate, blood pressure or oxygen saturation and transmits the information via wireless connection for example via Bluetooth to a mobile application. Charging of the body sensor is provided through at least one charging pad when the body sensor is placed in a charger.

The invention is based on the idea of providing a compact body sensor which comprises two separate parts each having a magnetic coupling part.

According to the invention the body sensor comprises a sensor part and a separate connecting part for securing the sensor part to other side of the user's body part such as an ear. In other words, the body sensor comprises two separate parts which are to be connected to opposite sides of the user's body part. The sensor part comprises a housing having a first surface and a second surface opposite to the first surface and the housing enclosing components of the sensor part inside the housing. The components of the sensor part comprise a circuit board, an optical sensor, a first magnetic coupling part, a transparent window and one or more other components. The circuit board is provided between the first surface of the housing and the second surface of the housing within the housing and preferably such that the circuit board extends substantially parallel to the first surface or to the second surface of the housing inside the housing. The optical sensor is configured to measure body activity, such as heart rate, blood pressure or oxygen saturation by optical measurement and the optical sensor is provided between the circuit board and the first side surface within the housing. In other words, the optical sensor is provided inside the housing in a place which is located between the first surface of the housing and the circuit board provided inside the housing. The transparent window is provided on the first surface of the housing and directly facing the optical sensor such that the transparent window is configured to allow light pass from the optical sensor through the first surface of the housing when the optical sensor is sending light to outside of the housing and receiving light from outside of the housing through the first surface of the housing to the optical sensor. The optical sensor and the transparent window are provided successively in the direction from the first surface toward the second surface. The transparent window and the optical sensor can be separate parts or the transparent window can be an integral part of the optical sensor. The first magnetic coupling part is provided in connection with the optical sensor such that the first magnetic coupling part provides an effective magnetic coupling with a second magnetic coupling part placed on the other side of the user's body part such as an ear so that the transparent window is provided firmly against user's body part such as an ear in order to allow the light from the optical sensor to reach the skin of the body part and measure the body activity such as heart rate according to the optical measurement. The sensor part further comprises one or more other components provided inside the housing. The separate connecting part is independent from the sensor part and comprises a second magnetic coupling part configured to provide a magnetic coupling with the first magnetic coupling part via the first surface of the housing for securing the sensor part to other side of the user's body part such as an ear.

In a preferable embodiment the first magnetic coupling part in the sensor part is a magnet and the second magnetic coupling part in the separate connecting part is a magnet. In another embodiment the first magnetic coupling part in the sensor part is a magnet and the second magnetic coupling part in the separate connecting part is made of magnetic material or comprises magnetic material such that the magnetic coupling is formed between the first and second magnetic coupling part. In a still other embodiment the first magnetic coupling part is made of magnetic material or comprises magnetic material such that the magnetic coupling is formed between the first magnetic coupling part and the second magnetic coupling part being a magnet.

The components of the sensor part are provided in the housing in the following order from the first surface toward the second surface: the transparent window, the optical sensor, the circuit board and the first magnetic coupling part.

In a preferred embodiment the optical sensor and the first magnetic coupling part are provided on opposite surfaces of the circuit board. The circuit board may be a two-sided circuit board and the optical sensor is connected to the first surface of the circuit board and the first magnetic coupling part is provided in connection with the second surface of the circuit board. The first surface of the circuit board is facing toward the first surface of the housing and/or the second surface of the circuit board is facing toward the second surface of the housing. The first magnetic coupling part may be in contact with the circuit board or in a proximity of the circuit board.

Alternatively, the circuit board is provided parallel to the first surface of the housing and the optical sensor and the first magnetic coupling part are provided on opposite surfaces of the circuit board.

In a preferred embodiment the optical sensor and the first magnetic coupling part are at least partly superposed in the direction from the first surface toward the second surface, or the optical sensor and the first magnetic coupling part are at least partly superposed in the direction from the optical sensor toward the second surface. This is a very advantageous embodiment since the magnetic coupling provided between the first magnetic coupling part and the second magnetic coupling part are provided such that the optical sensor is at least partly between said magnetic coupling parts and is therefore firmly placed against the user's body part.

In a further embodiment the first magnetic coupling part is provided concentrically with the optical sensor in the direction from the first surface toward the second surface. In this embodiment the optical sensor is the most firmly placed against the user's body part because it is directly in the effect field of the first magnetic coupling.

In another embodiment the components are provided in the housing in the following order from the first surface toward the second surface: the transparent window, the optical sensor and the circuit board, and further such that the first magnetic coupling part is provided at least partly superposed with the transparent window within the housing between the circuit board and the first surface

The other components of the sensor part further comprise a rechargeable battery configured to provide power to the sensor part or the other components of the sensor part further comprise a rechargeable battery configured to provide power to the sensor part and at least one charging pad connected to the battery for recharging the battery.

In an embodiment the at least one charging pad is arranged to extend from the second surface of the housing toward the first surface and in contact with the circuit board.

In an embodiment the rechargeable battery is arranged to form part of the first magnetic coupling part; or alternatively the rechargeable battery is arranged to form the first magnetic coupling part. This means that the first magnetic coupling part may be provided in connection with the rechargeable battery either as part of the rechargeable battery, for example as an additional casing of the rechargeable battery, or the rechargeable battery may be made of a magnetic material so that the rechargeable battery forms the first magnetic coupling part.

In an embodiment the other components of the sensor part are provided in parallel with the optical sensor; or alternatively the other components of the sensor part are provided in parallel with the first magnetic coupling part; or alternatively the other components of the sensor part are provided in parallel with the optical sensor and the first magnetic coupling part.

In an embodiment the optical sensor comprises a wireless communication component; or alternatively the circuit board comprises a wireless communication component.

In an embodiment the body sensor is an ear heart rate sensor for measuring heart rate activity from a user's ear, the heart rate sensor comprises an optical sensor configured to measure heart rate activity by optical heart rate measurement, and the separate connecting part is independent from the sensor part and comprises the second magnetic coupling part configured to provide the magnetic coupling with the first magnetic coupling part via the first surface of the housing for securing the sensor part to other side of the user's ear.

An advantage of the invention is that as the sensor part and the separate connecting part are provided as separate elements of the body sensor the placement of the body sensor is suitable for many kinds of body parts such as ears and especially earlobes or noses or palms and especially in an area between the forefinger and the thumb. Further advantage of the invention is that as the sensor part comprises a small number of components it can be made as thin as possible which means that there is a small lever arm in the sensor part, whereby the sensor part stays stable when placed on the user's body part. Another advantage of the invention is that the separate connecting part can be changed when other kind of magnetic coupling is needed, for example a more powerful magnetic coupling when the user is running and less powerful magnetic coupling when the user is sleeping. The invention is defined by the appended claims.

### BRIEF DESCRIPTION OF THE DRAWINGS

The invention is described in detail by means of specific embodiments with reference to the enclosed drawings, in which
Figure 1 shows an embodiment of a body sensor according to the invention;
Figure 2 shows another embodiment of a body sensor according to the invention;
Figure 3 shows still another embodiment of a body sensor according to the invention;
Figure 4 shows yet another embodiment of a body sensor according to the invention;
Figure 5 shows an embodiment of a body sensor according to the invention;
Figure 6 shows another embodiment of a body sensor according to the invention;
Figure 7 shows still another embodiment of a body sensor according to the invention;
Figure 8 shows an embodiment of a sensor part of a body sensor according to the invention; and
Figure 9 shows another embodiment of a sensor part of a body sensor according to the invention.

### DETAILED DESCRIPTION OF THE INVENTION

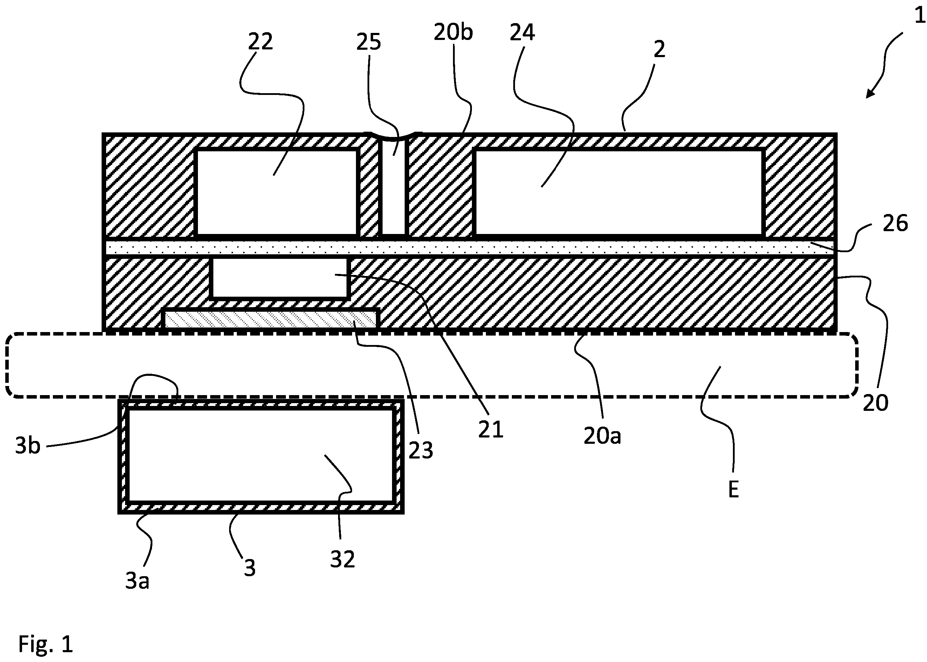

Figure 1 shows an embodiment of a body sensor 1 in which a sensor part 2 and a separate connecting part 3 are provided on opposite sides of the user's ear E. Although all the figures show an ear E as a body part the invention is not limited to the use of the body sensor 1 only in connection with an ear and therefore the ear E is represented as a dashed block in the figures. The sensor part 2 comprises a housing 20 having a first surface 20a placed against the ear E and a second surface 20b opposite to the first surface 20a. The housing 20 encloses components of the sensor part 2 inside the housing 20. The housing 20 in other words comprises two surfaces, the first surface 20a and the second surface 20b, such that the components of the sensor part 2 are provided between the surfaces 20a, 20b or such that the components are provided to extend from one surface toward the other surface. The housing 20 may comprise also side surfaces or alternatively the first surface 20a and the second surface 20b are configured such that they can be connected to each other providing a space between the first surface and the second surface where the components of the sensor part 2 are placed. The first surface 20a and the second surface 20b may have a curved shape or any other suitable shape for providing a housing 20. The components of the sensor part 2 are in this embodiment a circuit board 26, an optical sensor 21 configured to measure body activity by optical measurement, a first magnetic coupling part 22, a transparent window 23 provided on the first surface 20a of the housing 20 configured to allow light pass from the optical sensor 21 through the first surface 20a outside of the housing 20, a rechargeable battery 24 configured to provide power to the sensor part 2 and a charging pad 25 connected to the battery 24 for recharging the battery 24. The charging pad 25 extends from the second surface 20b of the housing 20 toward the first surface 20a. The components of the sensor part are provided in the housing 20 in the following order from the first surface toward the second surface: the transparent window 23, the optical sensor 21, the circuit board 26 and the first magnetic coupling part 22. In this embodiment the transparent window 23, the optical sensor 21 and the first magnetic coupling 22 are provided on top of each other in a transverse direction in relation to the first surface 20a such that the circuit board 26 is provided between the optical sensor 21 and the first magnetic coupling part 22. The optical sensor 21 is provided on the circuit board 26, or the optical sensor 21 is attached on the surface of the circuit board 26 such that the optical sensor 21 and the circuit board 26 form a compact assembly and the first magnetic coupling part 22 is provided on the other side of the circuit board 26 than the optical sensor 21 and preferably in a direct contact with the optical sensor 21 and the circuit board 26 assembly. In other words, the optical sensor 21 and the circuit board 26 form a uniform assembly and the first magnetic coupling part 22 is provided in connection with the assembly, preferably in a direct contact with the assembly. The transparent window 23, the optical sensor 21, the circuit board 26 and the first magnetic coupling 22 are arranged immediately successively in the housing 20 from the first surface 20a of the housing 20 toward the second surface 20b of the housing 20. Being arranged immediately successively in the housing 20 means that the transparent window 23, the optical sensor 21, the circuit board 26 and the first magnetic coupling 22 are provided either against each other or such that there is only structure of the housing between them or alternatively such that some of said transparent window 23, said optical sensor 21, said circuit board 26 and said first magnetic coupling 22 are against each other and some of them have the structure of the housing between them. In other words, the transparent window 23, the optical sensor 21, the circuit board 26 and the first magnetic coupling part 22 are provided in the housing such that the first magnetic coupling part 22, the optical sensor 23 and the transparent window 21 are provided along the same line from the first surface 20a of the housing 20 toward the second surface 20b of the housing 20, and such that when the second magnetic coupling part 32 of the separate connecting part 3 is forming a magnetic coupling with the first magnetic coupling part 22 of the sensor part 2 the transparent window 23, the optical sensor 21 and the circuit board 26 are provided between the first magnetic coupling part 22 and the second magnetic coupling part 32 and the magnetic coupling between the first magnetic coupling part 22 and the second magnetic coupling part 32 is provided through the transparent window 23, the optical sensor 21 and the circuit board 26.

The other components of the sensor part 2 are provided in this embodiment in parallel with the first magnetic coupling part 22 so that the rechargeable battery 24 and the charging pad 25 are provided next to the first magnetic coupling part 22. Other electronical components are provided in connection with the circuit board 26, which can be one-sided circuit board 26 or two-sided circuit board 26.

Figure 1 also shows a separate connecting part 3 which is independent from the sensor part 2 and comprises a second magnetic coupling part 32 configured to provide a magnetic coupling with the first magnetic coupling part 22 via the first surface 20a of the housing 20 for securing the sensor part 2 to other side of the user's ear. The separate connecting part 3 is preferably placed on the front side of the user's ear such that a back side 3b of the separate connecting part 3 is against the user's ear and a front side 3a of the separate connecting part 3 is visible. The front side 3a of the separate connecting part 3 may comprise jewellery or other surface structure. Alternatively, the whole separate connecting part 3 may comprise jewellery or be formed of one piece or multiple piece of jewellery.

The body sensor 1 comprises two separate parts: the sensor part 2 and the separate connecting part 3. When the body sensor 1 is used, i.e. in a use mode, the user's ear or other body part is placed between the first surface 20a of the housing 20 of the sensor part 2 and the separate connecting part 3 such that the magnetic coupling is provided between the first magnetic coupling part 22 and second magnetic coupling part 32 via the user's ear E or via some other body part.

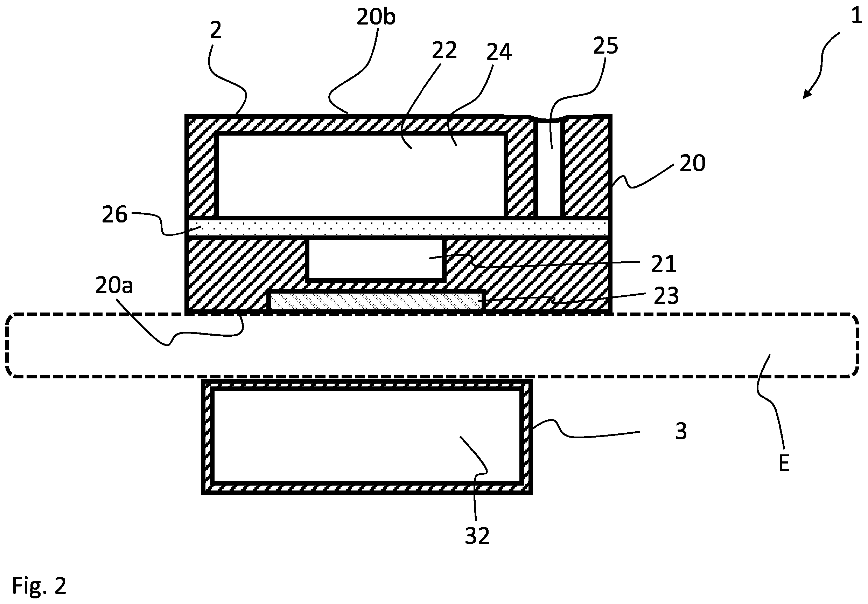

Figure 2 shows another embodiment of a body sensor 1 in which the transparent window 23, the optical sensor 21, the circuit board 26 and the first magnetic coupling 22 are provided on top of each other in a transverse direction in relation to the first surface 20a of the housing 20 similarly as shown in figure 1. The transparent window 23, the optical sensor 21, the circuit board 26 and the first magnetic coupling 22 are arranged immediately successively in the housing 20 similarly as shown in figure 1. In this embodiment the rechargeable battery 24 is arranged to form part of the first magnetic coupling part 22 or alternatively the rechargeable battery 24 is arranged to form the first magnetic coupling part 22. As the rechargeable battery 24 and the first magnetic coupling part 22 are combined in this embodiment the sensor part 2 is provided in a very compact form. The separate connecting part 3 shown in figure 2 is similar as shown in figure 1. In this embodiment when the second magnetic coupling part 32 of the separate connecting part 3 is forming a magnetic coupling with the first magnetic coupling part 22 of the sensor part 2 the transparent window 23, the optical sensor 21 and the circuit board 26 are provided between the first magnetic coupling part 22 and the second magnetic coupling part 32 and the magnetic coupling between the first magnetic coupling part 22 and the second magnetic coupling part 32 is provided through the transparent window 23, the optical sensor 21 and the circuit board 26 similarly as in connection with the figure 1 with the exception that the first magnetic coupling part 22 and the rechargeable battery 24 are combined as one.

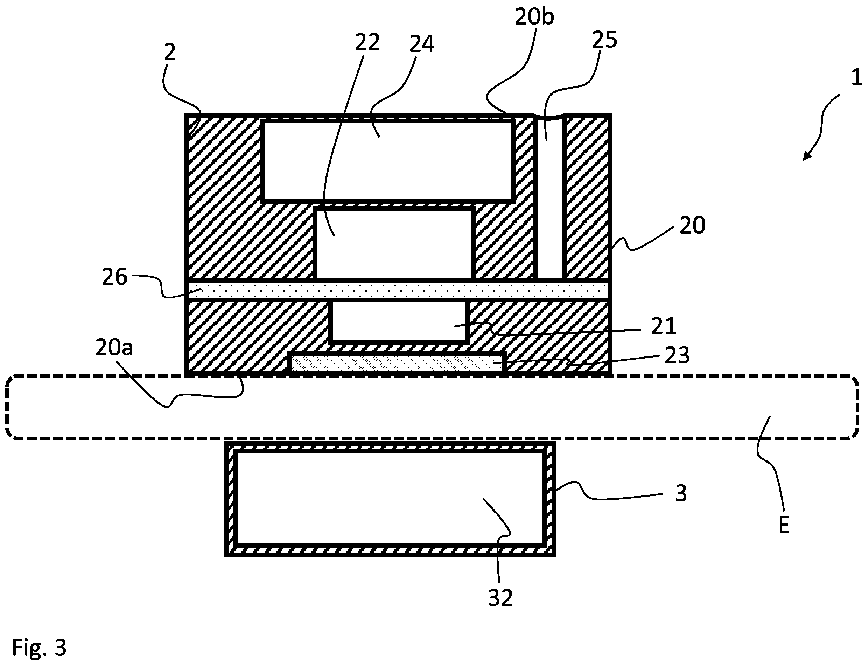

Figure 3 shows an embodiment of a body sensor in which the transparent window 23, the optical sensor 21, the circuit board 26 and the first magnetic coupling part 22 are provided in the housing 20 similarly as shown in figure 1 and the rechargeable battery 24 is provided between the first magnetic coupling part 22 and the second surface 20b of the housing 20. In this embodiment the magnetic coupling forming between the first magnetic coupling part 22 and the second magnetic coupling part 32 is provided through the transparent window 23, the optical sensor 21 and the circuit board 26.

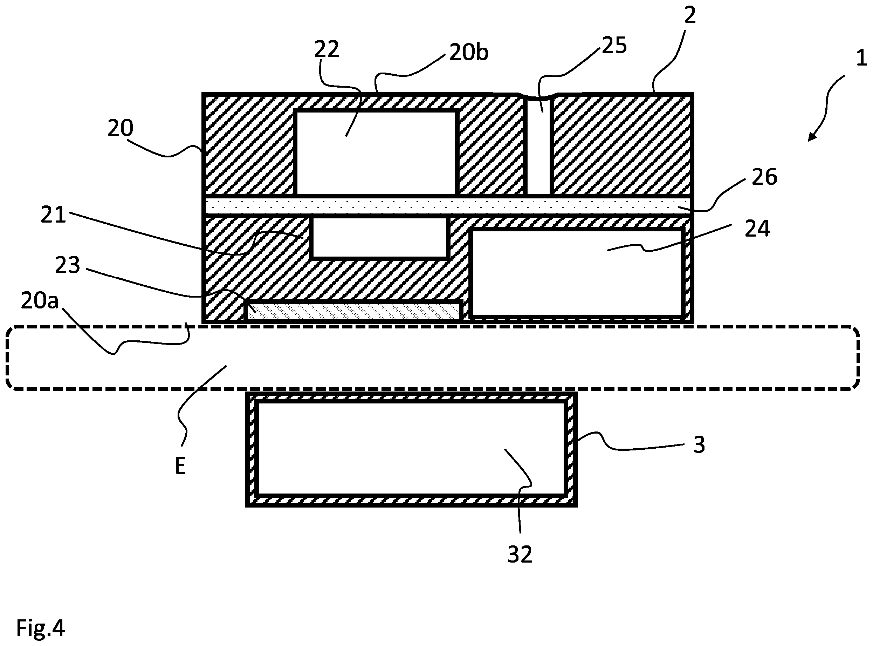

Figure 4 shows an embodiment in which the transparent window 23, the optical sensor 21, the circuit board 26 and the first magnetic coupling part 22 are provided inside the housing 20 similarly as in connection with figures 1-3. In this embodiment the rechargeable battery 24 is provided next to the transparent window and/or the optical sensor 21, i.e. on the same side of the circuit board 26 as the transparent window 23 and the optical sensor 21 in the housing 20.

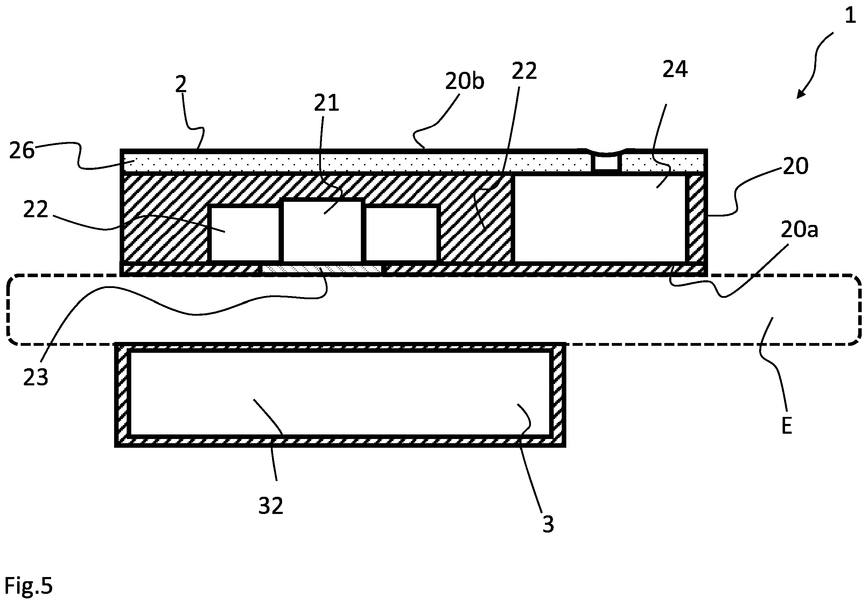

Figure 5 shows an embodiment of a sensor part 2 of a body sensor 1 in which the optical sensor 21 is provided within the first magnetic coupling part 22. The first magnetic coupling part 22 comprises a through hole suitable for the optical sensor 21 such that when the optical sensor 21 is provided in the through hole the first magnetic coupling part 22 surrounds the optical sensor 21. The first magnetic coupling part 22 having a through hole creates magnetic flux which operates similarly with regards to the first magnetic coupling part arranged in the through hole, as when the first magnetic coupling part 22 is provided at least partly superposed with the optical sensor 21 in a direction from the first surface 20a toward the second surface 20b. In other words, the first magnetic coupling part 22 having a through hole through the first magnetic coupling part which the through hole is such that the optical sensor 21 can be arranged in the through hole such that the first magnetic coupling part 22 surrounds the optical sensor 21 generates an equivalent magnetic flux through the optical sensor 21 as a first magnetic coupling part 22 arranged superposed with the optical sensor such that the optical sensor 21 is arranged between the transparent window 23 in the first surface 20a of the housing 20 and the first magnetic coupling part 22 in a direction from the first surface 20a toward the second surface 20b. In the embodiment shown in figure 5 the first magnetic coupling part 22 having a through hole is arranged partly in the area of the transparent window 23. The magnetic flux created by the first magnetic coupling part 22 goes through the optical sensor 21 which is arranged in the through hole in the middle of the first magnetic coupling part 22 such that the magnetic coupling between the first magnetic coupling part 22 and the second magnetic coupling part 32 provided on the opposite side of the ear E is equivalent to the magnetic coupling created between a first magnetic coupling part provided superposed to the optical sensor in a direction from the first surface 20a toward the second surface 20b and a second magnetic coupling part 32 of the separate connecting part 3 provided on the opposite side of the ear E.

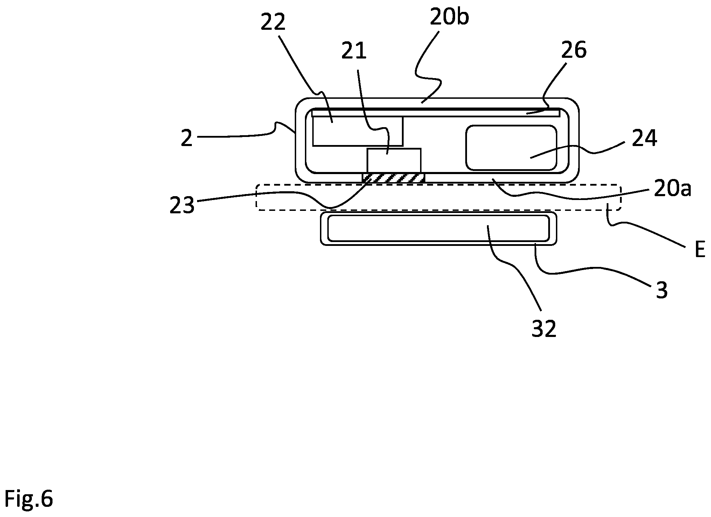

Figure 6 shows an embodiment of a sensor part 2 of a body sensor 1 in which the housing 20 of the sensor part 2 is made watertight such that the transparent window 23 forms part of the first surface 20a of the housing in a watertight manner. The optical sensor 21 is provided next to the transparent window 23 and the first magnetic coupling part 22 is arranged next to the optical sensor 21 in a direction from the first surface 20a to the second surface 20b. In other words, the optical sensor 21 is arranged between the first magnetic coupling part 22 and the transparent window 23 in the direction from the first surface 20a to the second surface 20b. In still other words, the first magnetic coupling part 22 is arranged at least partly superposed relative to the optical sensor 21 in the direction from the second surface 20b of the housing 20 toward the first surface 20a of the housing 20. Further the first magnetic coupling part 22 is arranged at least partly superposed with the optical sensor 21 and the transparent window 23 when viewed from the second surface 20b toward the first surface 20a. In other words, the magnetic flux generated by the first magnetic coupling part 22 affects through the optical sensor 21 and through the transparent window 23 forming part of the first surface 20a of the housing 20, such that the first magnetic coupling part 22 provides magnetic interaction with the second magnetic coupling part 32 of the separate connecting part 3 through the optical sensor 21 and the transparent window 23 as well as through the ear E. The sensor part 2 also comprises circuit board 26 and a battery 24 provided within the housing 20 similarly as described earlier.

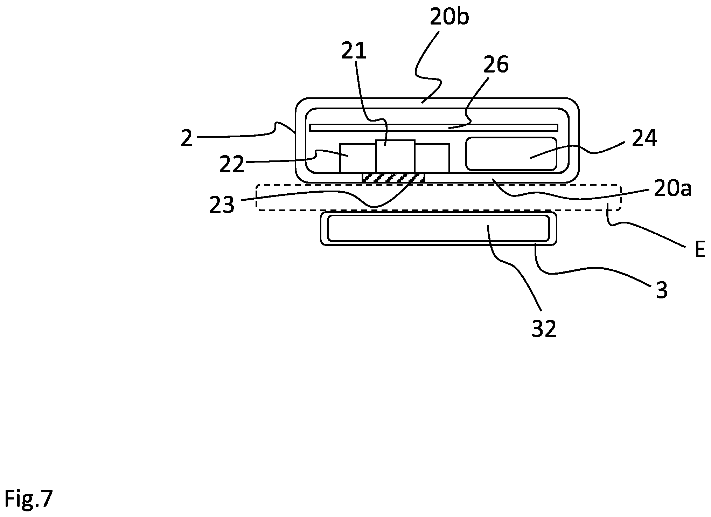

Figure 7 shows an embodiment of a sensor part 2 of a body sensor 1 and a separate connecting part 3 arranged on the opposite side of the ear E than the sensor part 2. The housing 20 of the sensor part 2 is made watertight such that the transparent window 23 forms part of the first surface 20a of the housing in a watertight manner similarly as in the embodiment shown in figure 6. Difference to the embodiment shown in figure 6 is the first magnetic coupling part 22 which in this embodiment is arranged to surround the optical sensor 21. In other words, the first magnetic coupling part 22 comprises a through hole in the first magnetic coupling part 22 in which the optical sensor 21 is provided such that the first magnetic coupling part 22 surrounds the optical sensor from the sides of the optical sensor 21. The through hole of the first magnetic coupling part 22 is arranged to go through the first magnetic coupling part 22 such that when the first magnetic coupling part 22 is arranged inside the housing 20 against the first surface 20a of the housing 20 or in the vicinity of the first surface 20a the through hole extends through the first magnetic coupling part 22 in the direction of the first surface 20a of the housing 20 to the second surface 20b of the housing 20 and such that the optical sensor 21 arranged in the through hole is against the transparent window 23 of the housing 20 or in the vicinity of the transparent window 23 of the housing 20. Other components of the sensor part 2 are provided inside the housing 20 according to what is described earlier. The magnetic flux generated by the first magnetic coupling part 22 provides magnetic coupling through the optical sensor 21 arranged within the first magnetic coupling part 22 and through the transparent window and through the ear E with the second magnetic coupling part 32 of the separate connecting part 3.

In the embodiments shown in the figures the optical sensor 21 and the first magnetic coupling part 22 are at least partly superposed in the direction from the first surface 20a toward the second surface 20b or the first magnetic coupling part 22 is provided concentrically with the optical sensor 21 in the direction from the first surface 20a toward the second surface 20b. This means that the magnetic flux generated by the first magnetic coupling part 22 affects through the optical sensor 21 so that the magnetic coupling between the first magnetic coupling part 22 and the second magnetic coupling part 32 provides a firm attachment between the user's ear E and the transparent window 23 through which the optical sensor 21 operates.

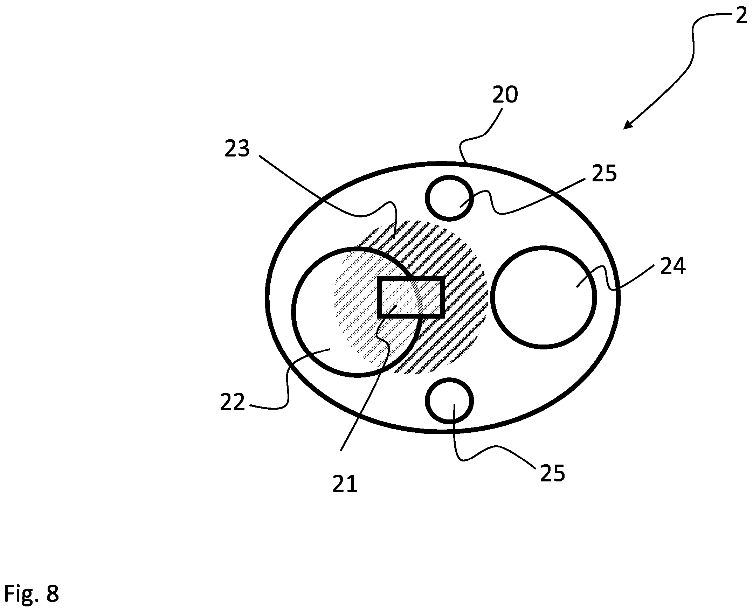

Figure 8 shows an embodiment of a sensor part 2 of a body sensor 1 in which the optical sensor 21, the transparent window 23 and the first magnetic coupling part 22 are directly next to each other within the structure of the housing 20 such that said optical sensor 21 and said transparent window 23 are placed directly on top of each other and the first coupling part 22 is placed such that part of the first coupling part is placed directly on top of the transparent window 23 and the optical sensor 21 and part of the first coupling part 22 is placed only on top of the transparent window 23. In other words, the optical sensor 21, the transparent window 23 and the first magnetic coupling part 22 are provided directly next to each other inside the housing in the direction from the first surface 20a toward the second surface 20b. The rechargeable battery 24 and the charging pads 25, as in this embodiment there are two charging pads 25, are placed next to the optical sensor 21, the transparent window 23 and the first magnetic coupling part 22 inside the housing.

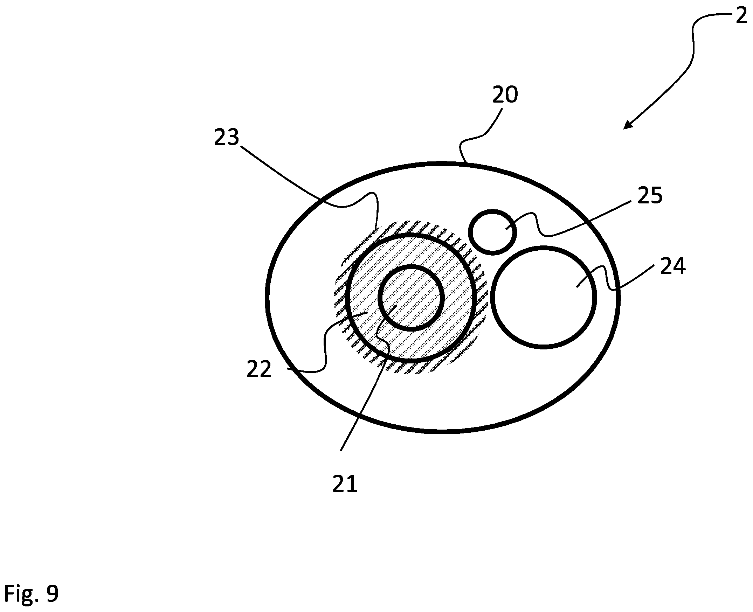

Figure 9 shows another embodiment of a sensor part 2 of a body sensor 1 in which the optical sensor 21, the transparent window 23 and the first magnetic coupling part 22 are placed directly next to each other within the structure of the housing 20 such that the optical sensor 21 is placed on top of the transparent window 23 and the first magnetic coupling part 22 is placed on top of the optical sensor 21 or alternatively the first magnetic coupling part 22 is placed on top of the transparent window 23 such that the optical sensor 21 is within the first magnetic coupling part 22. The optical sensor 21 being within the first magnetic coupling part 22 means for example such that the first magnetic coupling part 22 comprises a recess in which the optical sensor 21 is provided such that the first magnetic coupling part 22 surrounds the optical sensor 21 on its sides but such that the optical sensor is operable through the transparent window 23. In other words, a surface of the first magnetic coupling part 22 facing toward the transparent window 23 is preferably provided substantially on a same level as a surface of the optical sensor 21 facing toward the transparent window 23. Placing directly next to each other within the structure of the housing 20 means that the part can be placed directly next to each other without having any structures of the housing 20 between the parts or such that there is only structures of the housing 20 between some or all of the parts, however, no other components are provided between the optical sensor 21, the transparent window 23 and the first magnetic coupling part 22 or the transparent window 23 and the combination of the optical sensor 21 and the first magnetic coupling part 22. In the embodiment shown in figure 6 the optical sensor 21, the transparent window 23 and the first magnetic coupling part 22 are directly on top of each other such that the whole of the optical sensor 21 is on top of the transparent window 23 and the first magnetic coupling part 22 is placed to cover the optical sensor 21. Alternatively, the transparent window 23 and the combination of the optical sensor 21 and the first magnetic coupling part 22 are directly on top of each other such that the whole of the optical sensor 21 is on top of the transparent window 23 and the first magnetic coupling part 22 is provided around the optical sensor 21. The first magnetic coupling part 22 can then be a ring magnet arranged around the optical sensor 21. In this case, the first magnetic coupling part 22 surrounds the optical sensor 21 and looks similar as in figure 9 when viewed from above. The sensor part 2 shown in figures 10 and 11 are seen from above in a direction from the second surface 20b toward the first surface 20a.

The invention has been described above with reference to the examples shown in the figures. However, the invention is in no way restricted to the above examples but may vary within the scope of the claims.

## Claims

1. A body sensor (1) for measuring body activity of a user, the body sensor (1) comprises:
- a sensor part (2) comprising a housing (20) having a first surface (20a) and a second surface (20b) opposite to the first surface (20a), said housing (20) enclosing components of the sensor part (2) inside the housing (20), the components comprising:
a circuit board (26) provided between the first surface (20a) and the second surface (20b) within the housing (20),
an optical sensor (21) configured to measure body activity by optical measurement, the optical sensor (21) being provided between the circuit board (26) and the first surface (20a) within the housing (20),
a first magnetic coupling part (22),
a transparent window (23) provided on the first surface (20a) of the housing (20) and directly facing the optical sensor (21) such that the transparent window (23) is configured to allow light pass from the optical sensor (21) through the first surface (20a) to outside of the housing (20) and from outside of the housing through the first surface (20a) to the optical sensor (21), and
one or more other components provided inside the housing (20); **characterized in that** the first magnetic coupling part (22) is provided concentrically with the optical sensor (21) in the direction from the first surface (20a) toward the second surface (20b),
the components are provided in the housing (20) in the following order from the first surface toward the second surface: the transparent window (23), the optical sensor (21) and the circuit board (26), and further such that the first magnetic coupling part (22) is provided at least partly superposed with the transparent window (23) within the housing (20) between the circuit board (26) and the first surface (20a),
and
the body sensor (1) comprises:
- a separate connecting part (3) being independent from the sensor part (2) and comprising a second magnetic coupling part (32) configured to provide a magnetic coupling with the first magnetic coupling part (22) via the first surface (20a) of the housing (20) for securing the sensor part (2) to other side of a user's body part,
the sensor part (2) and the separate connecting part (3) are provided as separate elements of the body sensor (1).

2. A body sensor (1) according to claim 1, **characterized in that** the components are provided in the housing (20) in the following order from the first surface toward the second surface: the transparent window (23), the optical sensor (21), the circuit board (26) and the first magnetic coupling part (22).

3. A body sensor (1) according to claim 1 or 2, **characterized in that** the optical sensor (21) and the first magnetic coupling part (22) are provided on opposite surfaces of the circuit board (26); or
the circuit board (26) is provided parallel to the first surface (20a) of the housing (20) and the optical sensor (21) and the first magnetic coupling part (22) are provided on opposite surfaces of the circuit board (26).

4. A body sensor (1) according to any of claims 1 - 3, **characterized in that** the optical sensor (21) and the first magnetic coupling part (22) are at least partly superposed in the direction from the first surface (20a) toward the second surface (20b), or
the optical sensor (21) and the first magnetic coupling part (22) are at least partly superposed in the direction from the optical sensor (21) toward the second surface (20b).

5. A body sensor (1) according to claim 1, **characterized in that** the other components of the sensor part (2) further comprise a rechargeable battery (24) configured to provide power to the sensor part (2).

6. A body sensor (1) according to claim 5, **characterized in that** the other components of the sensor part (2) further comprise at least one charging pad (25) connected to the battery (24) for recharging of the battery (24).

7. A body sensor (1) according to claim 6, **characterized in that** the at least one charging pad (25) is arranged to extend from the second surface (20b) of the housing (20) toward the first surface (20a) and in contact with the circuit board (26).

8. A body sensor (1) according to any previous claim, **characterized in that** the other components of the sensor part (2) are provided in parallel with the optical sensor (21); or
the other components of the sensor part (2) are provided in parallel with the first magnetic coupling part (22); or
the other components of the sensor part (2) are provided in parallel with the optical sensor (21) and the first magnetic coupling part (22).

9. A body sensor (1) according to any previous claim, **characterized in that** the optical sensor (21) comprises a wireless communication component; or
the circuit board (26) comprises a wireless communication component.

10. A body sensor (1) according to any previous claim, **characterized in that** the body sensor (1) is an ear heart rate sensor for measuring heart rate activity from a user's ear, the heart rate sensor comprises an optical sensor (21) configured to measure heart rate activity by optical heart rate measurement, and
the separate connecting part (3) being independent from the sensor part (2) and comprising the second magnetic coupling part (32) configured to provide the magnetic coupling with the first magnetic coupling part (22) via the first surface (20a) of the housing (20) for securing the sensor part (2) to other side of the user's ear.

11. A body sensor (1) according to any previous claim, **characterized in that** the optical sensor (21) and the first magnetic coupling part (22) are at least partly superposed in the direction from the first surface (20a) toward the second surface (20b), or the first magnetic coupling part (22) is provided concentrically with the optical sensor (21) in the direction from the first surface (20a) toward the second surface (20b).

## Patentansprüche

1. Körpersensor (1) zum Messen von körperlicher Aktivität eines Benutzers, wobei der Körpersensor (1) umfasst:
- einen Sensorteil (2), der ein Gehäuse (20) umfasst, das eine erste Fläche (20a) und eine der ersten Fläche (20a) gegenüberliegende zweite Fläche (20b) aufweist, wobei das Gehäuse (20) Komponenten des Sensorteils (2) im Inneren des Gehäuses (20) umschließt, wobei die Komponenten umfassen:
eine Leiterplatte (26), die zwischen der ersten Fläche (20a) und der zweiten Fläche (20b) innerhalb des Gehäuses (20) vorgesehen ist,
einen optischen Sensor (21), der so konfiguriert ist, dass er körperliche Aktivität durch optische Messung misst, wobei der optische Sensor (21) zwischen der Leiterplatte (26) und der ersten Fläche (20a) innerhalb des Gehäuses (20) vorgesehen ist,
einen ersten magnetischen Kopplungsteil (22),
ein transparentes Fenster (23), das an der ersten Fläche (20a) des Gehäuses (20) vorgesehen ist und dem optischen Sensor (21) direkt zugewandt ist, derart, dass das transparente Fenster (23) so konfiguriert ist, dass es Licht vom optischen Sensor (21) durch die erste Fläche (20a) zur Außenseite des Gehäuses (20) und von der Außenseite des Gehäuses durch die erste Fläche (20a) zum optischen Sensor (21) durchlässt, und
eine oder mehrere andere Komponenten, die im Inneren des Gehäuses (20) vorgesehen sind; **dadurch gekennzeichnet, dass** der erste magnetische Kopplungsteil (22) konzentrisch mit dem optischen Sensor (21) in der Richtung von der ersten Fläche (20a) zur zweiten Fläche (20b) hin vorgesehen ist,
wobei die Komponenten im Gehäuse (20) in der folgenden Reihenfolge von der ersten Fläche zur zweiten Fläche hin vorgesehen sind: das transparente Fenster (23), der optische Sensor (21) und die Leiterplatte (26), und weiter derart, dass der erste magnetische Kopplungsteil (22) mindestens teilweise mit dem transparenten Fenster (23) überlagert innerhalb des Gehäuses (20) zwischen der Leiterplatte (26) und der ersten Fläche (20a) vorgesehen ist,
und
der Körpersensor (1) umfasst:
- einen separaten Verbindungsteil (3), der vom Sensorteil (2) unabhängig ist und einen zweiten magnetischen Kopplungsteil (32) umfasst, der so konfiguriert ist, dass er über die erste Fläche (20a) des Gehäuses (20) eine magnetische Kopplung mit dem ersten magnetischen Kopplungsteil (22) zum Sichern des Sensorteils (2) auf der anderen Seite eines Körperteils eines Benutzers bereitstellt,
wobei der Sensorteil (2) und der separate Verbindungsteil (3) als separate Elemente des Körpersensors (1) vorgesehen sind.

2. Körpersensor (1) nach Anspruch 1, **dadurch gekennzeichnet, dass** die Komponenten im Gehäuse (20) in der folgenden Reihenfolge von der ersten Fläche zur zweiten Fläche hin vorgesehen sind: das transparente Fenster (23), der optische Sensor (21), die Leiterplatte (26) und der erste magnetische Kopplungsteil (22).

3. Körpersensor (1) nach Anspruch 1 oder 2, **dadurch gekennzeichnet, dass** der optische Sensor (21) und der erste magnetische Kopplungsteil (22) an gegenüberliegenden Flächen der Leiterplatte (26) vorgesehen sind; oder
die Leiterplatte (26) parallel zur ersten Fläche (20a) des Gehäuses (20) vorgesehen ist und der optische Sensor (21) und der erste magnetische Kopplungsteil (22) an gegenüberliegenden Flächen der Leiterplatte (26) vorgesehen sind.

4. Körpersensor (1) nach einem der Ansprüche 1-3, **dadurch gekennzeichnet, dass** der optische Sensor (21) und der erste magnetische Kopplungsteil (22) in der Richtung von der ersten Fläche (20a) zur zweiten Fläche (20b) hin mindestens teilweise überlagert sind, oder
der optische Sensor (21) und der erste magnetische Kopplungsteil (22) in der Richtung vom optischen Sensor (21) zur zweiten Fläche (20b) hin mindestens teilweise überlagert sind.

5. Körpersensor (1) nach Anspruch 1, **dadurch gekennzeichnet, dass** die anderen Komponenten des Sensorteils (2) weiter eine aufladbare Batterie (24) umfassen, die so konfiguriert ist, dass sie dem Sensorteil (2) Strom bereitstellt.

6. Körpersensor (1) nach Anspruch 5, **dadurch gekennzeichnet, dass** die anderen Komponenten des Sensorteils (2) weiter mindestens ein mit der Batterie (24) verbundenes Ladepad (25) zum Aufladen der Batterie (24) umfassen.

7. Körpersensor (1) nach Anspruch 6, **dadurch gekennzeichnet, dass** das mindestens eine Ladepad (25) so angeordnet ist, dass es sich von der zweiten Fläche (20b) des Gehäuses (20) zur ersten Fläche (20a) hin erstreckt und mit der Leiterplatte (26) in Kontakt steht.

8. Körpersensor (1) nach einem vorstehenden Anspruch, **dadurch gekennzeichnet, dass** die anderen Komponenten des Sensorteils (2) parallel zum optischen Sensor (21) vorgesehen sind; oder
die anderen Komponenten des Sensorteils (2) parallel zum ersten magnetischen Kopplungsteil (22) vorgesehen sind; oder
die anderen Komponenten des Sensorteils (2) parallel zum optischen Sensor (21) und dem ersten magnetischen Kopplungsteil (22) vorgesehen sind.

9. Körpersensor (1) nach einem vorstehenden Anspruch, **dadurch gekennzeichnet, dass** der optische Sensor (21) eine Drahtlos-Kommunikationskomponente umfasst; oder
die Leiterplatte (26) eine Drahtlos-Kommunikationskomponente umfasst.

10. Körpersensor (1) nach einem vorstehenden Anspruch, **dadurch gekennzeichnet, dass** der Körpersensor (1) ein Ohr-Herzfrequenzsensor zum Messen von Herzfrequenzaktivität am Ohr eines Benutzers ist, wobei der Herzfrequenzsensor einen optischen Sensor (21) umfasst, der so konfiguriert ist, dass er Herzfrequenzaktivität durch optische Herzfrequenzmessung misst, und
wobei der separate Verbindungsteil (3) vom Sensorteil (2) unabhängig ist und den zweiten magnetischen Kopplungsteil (32) umfasst, der so konfiguriert ist, dass er über die erste Fläche (20a) des Gehäuses (20) die magnetische Kopplung mit dem ersten magnetischen Kopplungsteil (22) zum Sichern des Sensorteils (2) auf der anderen Seite des Ohrs des Benutzers bereitstellt.

11. Körpersensor (1) nach einem vorstehenden Anspruch, **dadurch gekennzeichnet, dass** der optische Sensor (21) und der erste magnetische Kopplungsteil (22) in der Richtung von der ersten Fläche (20a) zur zweiten Fläche (20b) hin mindestens teilweise überlagert sind oder der erste magnetische Kopplungsteil (22) in der Richtung von der ersten Fläche (20a) zur zweiten Fläche (20b) hin konzentrisch mit dem optischen Sensor (21) vorgesehen ist.

## Revendications

1. Capteur corporel (1) pour mesurer une activité corporelle d'un utilisateur, le capteur corporel (1) comprend :
- une partie capteur (2) comprenant un boîtier (20) présentant une première surface (20a) et une seconde surface (20b) opposée à la première surface (20a), ledit boîtier (20) renfermant des composants de la partie capteur (2) à l'intérieur du boîtier (20), les composants comprenant :
une carte de circuit imprimé (26) disposée entre la première surface (20a) et la seconde surface (20b) à l'intérieur du boîtier (20),
un capteur optique (21) configuré pour mesurer une activité corporelle par une mesure optique, le capteur optique (21) étant disposé entre la carte de circuit imprimé (26) et la première surface (20a) à l'intérieur du boîtier (20),
une première partie de couplage magnétique (22),
une fenêtre transparente (23) disposée sur la première surface (20a) du boîtier (20) et faisant directement face au capteur optique (21) de telle sorte que la fenêtre transparente (23) soit configurée pour permettre à la lumière de passer du capteur optique (21) à travers la première surface (20a) vers l'extérieur du boîtier (20) et de l'extérieur du boîtier à travers la première surface (20a) vers le capteur optique (21), et
un ou plusieurs autres composants disposés à l'intérieur du boîtier (20) ; **caractérisé en ce que** la première partie de couplage magnétique (22) est disposée de manière concentrique avec le capteur optique (21) dans la direction allant de la première surface (20a) vers la seconde surface (20b),
les composants sont disposés dans le boîtier (20) dans l'ordre suivant, de la première surface vers la seconde surface : la fenêtre transparente (23), le capteur optique (21) et la carte de circuit imprimé (26) et en outre de telle sorte que la première partie de couplage magnétique (22) soit disposée au moins partiellement superposée à la fenêtre transparente (23) à l'intérieur du boîtier (20) entre la carte de circuit imprimé (26) et la première surface (20a),
et
le capteur corporel (1) comprend :
- une partie de connexion distincte (3) qui est indépendante de la partie capteur (2) et comprenant une seconde partie de couplage magnétique (32) configurée pour fournir un couplage magnétique à la première partie de couplage magnétique (22) via la première surface (20a) du boîtier (20) pour fixer la partie capteur (2) à un autre côté d'une partie du corps d'un utilisateur,
la partie capteur (2) et la partie de connexion distincte (3) sont disposées en tant qu'éléments distincts du capteur corporel (1).

2. Capteur corporel (1) selon la revendication 1, **caractérisé en ce que** les composants sont disposés dans le boîtier (20) dans l'ordre suivant, de la première surface vers la seconde surface : la fenêtre transparente (23), le capteur optique (21), la carte de circuit imprimé (26) et la première partie de couplage magnétique (22).

3. Capteur corporel (1) selon la revendication 1 ou 2, **caractérisé en ce que** le capteur optique (21) et la première partie de couplage magnétique (22) sont disposés sur des surfaces opposées de la carte de circuit imprimé (26) ; ou
la carte de circuit imprimé (26) est disposée parallèlement à la première surface (20a) du boîtier (20), et le capteur optique (21) et la première partie de couplage magnétique (22) sont disposés sur des surfaces opposées de la carte de circuit imprimé (26).

4. Capteur corporel (1) selon l'une quelconque des revendications 1-3, **caractérisé en ce que** le capteur optique (21) et la première partie de couplage magnétique (22) sont au moins partiellement superposés dans la direction allant de la première surface (20a) vers la seconde surface (20b), ou
le capteur optique (21) et la première partie de couplage magnétique (22) sont au moins partiellement superposés dans la direction allant du capteur optique (21) vers la seconde surface (20b).

5. Capteur corporel (1) selon la revendication 1, **caractérisé en ce que** les autres composants de la partie capteur (2) comprennent en outre une pile rechargeable (24) configurée pour fournir de l'énergie à la partie capteur (2).

6. Capteur corporel (1) selon la revendication 5, **caractérisé en ce que** les autres composants de la partie capteur (2) comprennent en outre au moins un plot de charge (25) connecté à la pile (24) pour recharger la pile (24).

7. Capteur corporel (1) selon la revendication 6, **caractérisé en ce que** le au moins un plot de charge (25) est agencé pour s'étendre depuis la seconde surface (20b) du boîtier (20) vers la première surface (20a) et en contact avec la carte de circuit imprimé (26).

8. Capteur corporel (1) selon une quelconque revendication précédente, **caractérisé en ce que** les autres composants de la partie capteur (2) sont disposés en parallèle avec le capteur optique (21) ; ou
les autres composants de la partie capteur (2) sont disposés en parallèle avec la première partie de couplage magnétique (22) ; ou
les autres composants de la partie capteur (2) sont disposés en parallèle avec le capteur optique (21) et la première partie de couplage magnétique (22).

9. Capteur corporel (1) selon une quelconque revendication précédente, **caractérisé en ce que** le capteur optique (21) comprend un composant de communication sans fil ; ou
la carte de circuit imprimé (26) comprend un composant de communication sans fil.

10. Capteur corporel (1) selon une quelconque revendication précédente, **caractérisé en ce que** le capteur corporel (1) est un capteur de fréquence cardiaque auriculaire permettant de mesurer l'activité de fréquence cardiaque à partir de l'oreille d'un utilisateur, le capteur de fréquence cardiaque comprend un capteur optique (21) configuré pour mesurer l'activité de fréquence cardiaque par une mesure optique de fréquence cardiaque, et
la partie de connexion distincte (3) qui est indépendante de la partie capteur (2) et comprenant la seconde partie de couplage magnétique (32) configurée pour fournir le couplage magnétique à la première partie de couplage magnétique (22) via la première surface (20a) du boîtier (20) pour fixer la partie capteur (2) à l'autre côté de l'oreille de l'utilisateur.

11. Capteur corporel (1) selon une quelconque revendication précédente, **caractérisé en ce que** le capteur optique (21) et la première partie de couplage magnétique (22) sont au moins partiellement superposés dans la direction allant de la première surface (20a) vers la seconde surface (20b), ou la première partie de couplage magnétique (22) est disposée de manière concentrique avec le capteur optique (21) dans la direction allant de la première surface (20a) vers la seconde surface (20b).
